# EUROPEAN PATENT APPLICATION

(11) **EP 3 674 405 A1**
(43) Date of publication of application: **01.07.2020**
(21) Application number: 18848917.3
(22) Date of filing: 23.08.2018
(51) Int. Cl.: C12N 15/10, C12N 15/12, C12Q 1/6886

(54) **METHOD FOR ASSESSING RISK OF HEPATOCELLULAR CARCINOMA**

(30) Priority: 23.08.2017 JP 2017160284
(71) Applicant: National Cancer Center, Tokyo 104-0045 (JP); Sekisui Medical Co., Ltd., Tokyo 103-0027 (JP)
(72) Inventor: KANAI, Yae, Tokyo 1608582 (JP); ARAI, Eri, Tokyo 1608582 (JP); YOTANI, Takuya, Tokyo 103-0027 (JP); SUNAMURA, Ei-ichiro, Tokyo 103-0027 (JP)
(74) Representative: Blodig, Wolfgang
(86) International application number: PCT/JP2018/031092
(87) International publication number: WO 2019/039532

(57) **Abstract**

It is intended to provide highly sensitive and specific, rapid, and convenient method for evaluating a risk of hepatocellular carcinoma. The method for evaluating a risk of hepatocellular carcinoma comprises: (1) amplifying bisulfite-treated DNA derived from a liver tissue of a subject, wherein the DNA comprises a CpG site of an exon region of MGRN1 gene; (2) subjecting the obtained amplification product to ion exchange chromatography; and (3) determining whether or not the DNA is DNA obtained from a subject having a high risk of development of hepatocellular carcinoma on the basis of a peak shape of a detection signal of the chromatography.

SEQUENCE LISTING

<110>  National Cancer Center
       Sekisui Medical Co., Ltd.

<120>  Method for Evaluating Risk of Hepatocellular Carcinoma

<130>  DC0112

<150>  JP 2017-160284
<151>  2017-08-23

<160>  9
<170>  PatentIn version 3.1

<210>  1
<211>  283
<212>  DNA
<213>  Homo sapiens
<400>  1
tactggagaa gcgggctgtg tccacatagc cacctccacg gcccctcagc ctggcagggg      60
aggaggcagc ctccgcagat ggggccgctg acccgctgcc tttctctcca ccgcctgggg     120
taggtacaaa gacgatgccg acagccccac cgaggacggc gacaagcccc gggtgctcta     180
cagcctggag ttcaccttcg acgccgatgc ccgcgtggcc atcaccatct actgccaggc     240
atcggaggag ttcctgaacg gcagggcagt gtgagtcccg cgg                       283

<210>  2
<211>  208
<212>  DNA
<213>  Homo sapiens
<400>  2
agctggccct gcgggaaagc agctcccctg aggtgaggcc cccccgggga agctttgcgc      60
acccgcccgg gccagccctc ctccagcttc tgtcgctgga atcagacccc atcccactgc     120
ccgcctgggc gcccccgtgc ttgttggctc ttgctgagct gcgcggctcc ttagcctcgg     180
tctgtggagg cagcaccccc tggtggag                                        208

<210>  3
<211>  300
<212>  DNA
<213>  Homo sapiens
<400>  3
caggccctac agttaggagg gcagggcgtg gcgctgcagg cctgtgtgca gtgtggtgga      60
cctgcccctg cccgggaggt gccgggtgca gcgtgggcgc tgctgtggcg ggctgagttc     120
caggaccttc tcctggctcc ctgtgcacgt cgcagcgagg ccgtgtgggg gtgtgtgtga     180
atgtgcacgt gtgcgcgcgc atgtgagtgc gcacgtgagt gagtgtgagt atgcatgcgt     240
gtgtgtgccc ctgtgtcacc gggctctgct tgtgactgcg gaacctgctg ggtgctccgc     300

<210>  4
<211>  27
<212>  DNA
<213>  Artificial
<220>
<223>  Artificially synthesized primer sequence
<400>  4
tattggagaa gagggttgtg tttatat                                          27

<210>  5
<211>  23
<212>  DNA
<213>  Artificial
<220>
<223>  Artificially synthesized primer sequence
<400>  5

cccccaaact cacactaccc tac                                          23

<210>  6
<211>  23
<212>  DNA
<213>  Artificial
<220>
<223>  Artificially synthesized primer sequence
<400>  6
agttggtttt gagggaaagt agt                                          23

<210>  7
<211>  23
<212>  DNA
<213>  Artificial
<220>
<223>  Artificially synthesized primer sequence
<400>  7
ctccaccaaa aaatactacc tcc                                          23

<210>  8
<211>  25
<212>  DNA
<213>  Artificial
<220>
<223>  Artificially synthesized primer sequence
<400>  8
taggttttat agttaggagg gtagg                                        25

<210>  9
<211>  20
<212>  DNA
<213>  Artificial
<220>
<223>  Artificially synthesized primer sequence
<400>  9
cccaaacacc caacaaattc                                              20

## Description

### Field of the Invention

The present invention relates to a method for evaluating a risk of hepatocellular carcinoma by use of the detection of methylated DNA.

### Background of the Invention

Hepatocellular carcinoma (HCC) is a malignant tumor known worldwide, and a primary development factor thereof has been found to be infection by hepatitis viruses. The hepatitis viruses serving as the development factor of hepatocellular carcinoma are typically hepatitis B virus (HBV) and hepatitis C virus (HCV). HCC usually develops in patients having chronic hepatitis or liver cirrhosis associated with hepatitis virus infection. Since most of the patients already have a reduced hepatic function at the stage where HCC has developed, favorable treatment results cannot be expected as long as the cancer is diagnosed early. Hence, the surveillance (follow-up) of a precancerous condition such as chronic hepatitis or liver cirrhosis should be conducted as a priority. Particularly, HCC in patients having a high risk of development of HCC should be detected early by close surveillance and treated even if the patients notice no symptoms. On the other hand, such close surveillance places excessive burdens on patients having no risk of development of HCC. Therefore, the evaluation of a risk of development of HCC is very important for appropriately managing patients having a precancerous condition such as chronic hepatitis or liver cirrhosis.

Change in DNA methylation is one of the most generally observed epigenetic changes caused by carcinogenesis. It has been suggested that the change in DNA methylation is involved in early cancer and a precancerous stage. It has been reported that the splicing of DNA methyltransferase and/or change in DNA methylation associated with abnormal expression occurs in liver tissues found to have chronic hepatitis or liver cirrhosis obtained from HCC patients.

According to studies, the present inventor has previously proposed a method for evaluating a risk of HCC in a patient by detecting a DNA methylation level of a particular CpG site of genomic DNA in a liver tissue by pyrosequencing, mass spectrometry, or the like, and comparing the detected DNA methylation level with a cutoff value for determining noncancerous liver tissue samples (Patent Literature 1). This method achieves highly sensitive and specific evaluation of a risk of HCC. However, there is a strong demand for a method which can more efficiently evaluate a risk of HCC at lower cost while insuring high sensitivity and specificity.

A method for detecting methylated DNA by subjecting sample DNA treated with bisulfite to ion exchange chromatography has been disclosed recently (Patent Literature 2). The present inventors have further exploited this principle and proposed a method for determining the prognosis of renal cell carcinoma on the basis of a retention time obtained by subjecting sample DNA treated with bisulfite to ion exchange chromatography (Patent Literature 3).

### Citation List

### Patent Literature

Patent Literature 1: WO 2012/102377
Patent Literature 2: WO 2014/136930
Patent Literature 3: WO 2015/129916

### Summary of the Invention

### Problem to be solved by the Invention

A method for evaluating a risk of hepatocellular carcinoma (HCC) on the basis of the detection of a DNA methylation level using pyrosequencing or the like (e.g., Patent Literature 1) is highly sensitive and specific, but unfortunately requires time, labor, and cost for detecting the DNA methylation level. An object of the present invention is to provide a convenient and rapid method for evaluating a risk of HCC at low cost while having high sensitivity and specificity.

### Means for solving the Invention

The present inventors have found that the DNA methylation of a CpG site can be detected conveniently and rapidly by treating, with bisulfite, sample DNA comprising the CpG site obtained from a liver tissue of a subject, amplifying the DNA, and separating the amplification product by ion exchange chromatography. The present inventors have further found that a peak shape of a detection signal obtained by the chromatography differs between a high-risk group and a low-risk group of HCC. The peak shape of a detection signal obtained by the chromatography serves as an index for evaluating a risk of HCC.

Further surprisingly, the present inventors have found that in the risk evaluation of HCC by the DNA methylation analysis of a CpG site using the ion exchange chromatography, a particular CpG site used as an analyte can achieve remarkably high sensitivity and specificity as compared with other CpG sites. Thus, the combination of the DNA methylation analysis using the ion exchange chromatography with use of the particular CpG site as a sample achieves the risk evaluation of HCC having all of convenience, rapidness, and high sensitivity and specificity.

Accordingly, the present invention provides the followings:
[1] A method for evaluating a risk of hepatocellular carcinoma, comprising:
   (1) amplifying bisulfite-treated DNA derived from a liver tissue of a subject, wherein the DNA comprises a CpG site of an exon region of MGRN1 gene;
   (2) subjecting the obtained amplification product to ion exchange chromatography; and
   (3) determining whether or not the DNA is DNA obtained from a subject having a high risk of development of hepatocellular carcinoma on the basis of a peak shape of a detection signal of the chromatography.
[2] A method for evaluating a risk of hepatocellular carcinoma, comprising:
   (1) amplifying bisulfite-treated DNA derived from a liver tissue of a subject, wherein the DNA comprises a CpG site of an exon region of MGRN1 gene;
   (2) subjecting the obtained amplification product to ion exchange chromatography; and
   (3) determining whether or not the subject has a high risk of development of hepatocellular carcinoma on the basis of a peak shape of a detection signal of the chromatography.
[3] The method according to [1] or [2], wherein the DNA comprises DNA consisting of the nucleotide sequence represented by SEQ ID NO: 1, or a nucleotide sequence having at least 95% identity to the sequence.
[4] The method according to any one of [1] to [3], wherein in the step (3), when the peak shape of a detection signal is a unimodal peak of methylated DNA, the DNA is selected as DNA obtained from a subject having a high risk of development of hepatocellular carcinoma, and when the peak shape of a detection signal is bimodal, the DNA is selected as DNA obtained from a subject having a low risk of development of hepatocellular carcinoma.
[5] The method according to [4], wherein
   the step (3) comprises comparing the retention time of the peak of the detection signal with the retention time of a peak of a detection signal of a positive control or a negative control to confirm that the peak of the methylated DNA has been obtained, wherein
   the detection signal of the positive control is obtained by subjecting, to ion exchange chromatography, DNA obtained by the bisulfite treatment and amplification of 100% methylated DNA consisting of the same sequence as that of the DNA derived from a liver tissue of a subject, and
   the detection signal of the negative control is obtained by subjecting, to ion exchange chromatography, DNA obtained by the bisulfite treatment and amplification of unmethylated DNA consisting of the same sequence as that of the DNA derived from a liver tissue of a subject.
[6] The method according to any one of [1] to [5], wherein the ion exchange chromatography is anion exchange chromatography.

### Effects of the Invention

According to the present invention, a risk of hepatocellular carcinoma can be evaluated more conveniently and rapidly while the sensitivity and specific of detection are maintained, as compared with a conventional method using a DNA methylation level detected by pyrosequencing or the like as an index (e.g.,

### Patent Literature 1).

### Brief Description of the Drawings

[Figure 1] Figure 1 shows exemplary chromatograms obtained from a Liv25 region in noncancerous liver tissue samples (N group) derived from HCC patients. Each diagram shows data from the individual sample, and the symbol in each diagram denotes sample ID.
[Figure 2] Figure 2 shows plots of first derivative values of the data of Figure 1.
[Figure 3] Figure 3 shows exemplary chromatograms obtained from the Liv25 region in healthy liver tissue samples (C group). Each diagram shows data from the individual sample, and the symbol in each diagram denotes sample ID.
[Figure 4] Figure 4 shows plots of first derivative values of the data of Figure 3.

### Embodiments for carrying out the Invention

In the present specification, the "hepatocellular carcinoma" (also referred to as HCC) means primary liver cancer which develops from hepatocytes serving as the parenchyma of the liver.

In the present specification, the "risk of hepatocellular carcinoma" means a risk of development of hepatocellular carcinoma.

In the present specification, the "CpG site" means a site where a phosphodiester bond (p) is formed between cytosine (C) and guanine (G) on the DNA sequence.

In the present specification, the "DNA methylation" means a state where carbon at position 5 of cytosine is methylated at the CpG site.

In the present specification, the "methylation level" of DNA means the proportion of methylation of the DNA (also referred to as a methylation rate).

In the present specification, the "at least 95% identity" as to a nucleotide sequence refers to 95% or higher, preferably 97% or higher, more preferably 98% or higher, further preferably 99% or higher, still further preferably 99.5% or higher identity.

In one embodiment, the present invention provides a method for evaluating a risk of HCC, comprising:
(1) amplifying bisulfite-treated DNA derived from a liver tissue of a subject, wherein the DNA comprises a CpG site of an exon region of MGRN1 gene;
(2) subjecting the obtained amplification product to ion exchange chromatography; and
(3) determining whether or not the DNA is DNA obtained from a subject having a high risk of development of HCC on the basis of a peak shape of a detection signal of the chromatography.

In another embodiment, the present invention provides a method for evaluating a risk of HCC, comprising:
(1) amplifying bisulfite-treated DNA derived from a liver tissue of a subject, wherein the DNA comprises a CpG site of an exon region of MGRN1 gene;
(2) subjecting the obtained amplification product to ion exchange chromatography; and
(3) determining whether or not the subject is a subject having a high risk of development of HCC on the basis of a peak shape of a detection signal of the chromatography.

In the present invention, DNA comprising a CpG site of an exon region of MGRN1 gene derived from a liver tissue obtained from a subject (hereinafter, also referred to as sample DNA in the present specification) is treated with bisulfite and subsequently amplified.

Examples of the "subject" according to the present invention include, but are not particularly limited to, healthy individuals as well as hepatitis B infected individuals, hepatitis C infected individuals, chronic hepatitis patients, liver cirrhosis patients, hepatocellular carcinoma patients and individuals suspected thereof. Preferably, the subject is a human having hepatitis B, hepatitis C, chronic hepatitis or liver cirrhosis, and such a subject is generally known as an individual likely to develop hepatocellular carcinoma.

The method for preparing the "DNA derived from a liver tissue" used in the present invention is not particularly limited, and an approach known in the art can be appropriately selected for use. Examples of the method known in the art for preparing genomic DNA include phenol-chloroform method (method of treating the liver tissue with a proteolytic enzyme (proteinase K), a surfactant (SDS), and phenol to denature proteins in the tissue, and subsequently precipitating and extracting DNA from the tissue with chloroform, ethanol, or the like), and DNA extraction method using a commercially available DNA extraction kit, for example, QIAamp DNA Mini kit (manufactured by Qiagen N.V.), Clean Columns (manufactured by Hermes-NexTec GmbH), AquaPure (manufactured by Bio-Rad Laboratories, Inc.), ZR Plant/Seed DNA Kit (manufactured by Zymo Research Corp.), prepGEM (manufactured by ZyGEM NZ, Ltd.), or BuccalQuick (manufactured by TrimGen Corp.).

Examples of the liver tissue from which genomic DNA is prepared by such a method include, but are not particularly limited to, liver tissues themselves collected upon biopsy or the like, frozen liver tissues, and liver tissues fixed in formalin or embedded in paraffin. A frozen liver tissue is desirably used from the viewpoint of suppressing the degradation of the genomic DNA in the tissue. In the present invention, the status (the stages of chronic hepatitis and liver cirrhosis, hepatitis virus infection, inflammation or fibrosis, etc.) of the liver tissue for use in the preparation of the genomic DNA, and its distance from a focus of hepatocellular carcinoma are not particularly limited.

The "CpG site of an exon region of MGRN1 gene" according to the present invention refers to a CpG site of an exon region of human MGRN1 (mahogunin ring finger 1) gene represented by NCBI Gene ID: 23295 ([www.ncbi.nlm.nih.gov/gene/23295]).

The sample DNA used in the present invention is DNA comprising a CpG site of an exon region of MGRN1 gene. The sample DNA is, for example, DNA comprising a CpG site positioned in an upstream region of the MGRN1 gene exon. Examples of such sample DNA include DNA consisting of the nucleotide sequence represented by SEQ ID NO: 1. Alternatively, MGRN1 gene exon region-derived DNA consisting of a nucleotide sequence having at least 95% identity to the nucleotide sequence represented by SEQ ID NO: 1 is also an example of the sample DNA of the present invention. Preferably, the sample DNA comprises the nucleotide sequence represented by SEQ ID NO: 1, or DNA consisting of a nucleotide sequence having at least 95% identity to the sequence, and is more preferably DNA consisting of the nucleotide sequence represented by SEQ ID NO: 1.

The method for treating the sample DNA with bisulfite is not particularly limited, and an approach known in the art can be appropriately selected for use. Examples of the method known in the art for bisulfite treatment include methods as mentioned below using a commercially available kit, for example, EpiTect Bisulfite Kit (48) (manufactured by Qiagen N.V.), MethylEasy (manufactured by Human Genetic Signatures Pty), Cells-to-CpG Bisulfite Conversion Kit (manufactured by Applied Biosystems, Inc.), or CpGenome Turbo Bisulfite Modification Kit (manufactured by Merck Millipore).

Examples of the method for amplifying the bisulfite-treated sample DNA include, but are not particularly limited to, arbitrary nucleic acid amplification methods such as PCR. The conditions for amplification reaction are not particularly limited, and an approach known in the art can be appropriately selected for use according to the sequence, length, amount, etc. of the DNA to be amplified. Preferably, the DNA is amplified by PCR. The chain length of the amplification product can be appropriately adjusted in consideration of factors such as reduction in amplification reaction time and reduction in analysis time in ion exchange chromatography, and maintenance of separation performance. For example, the chain length of the PCR amplification product is preferably 1,000 bp or shorter, more preferably 500 bp or shorter, further preferably 300 bp or shorter. On the other hand, the chain length of the PCR amplification product is preferably 31 bp or longer, more preferably 40 bp or longer, because the chain length of primers for PCR is preferably 15 mer or longer in order to avoid nonspecific amplification.

Preferred examples of the primer set for the PCR amplification of the DNA according to the present invention include a primer set represented by SEQ ID NOs: 4 and 5. Preferred examples of the conditions for the PCR include, but are not limited to, [94°C for 1 min → 64°C for 1 min → 72°C for 1 min] × 5 cycles → [94°C for 1 min → 62°C for 1 min → 72°C for 1 min] × 5 cycles × [94°C for 1 min → 60°C for 1 min → 72°C for 1 min] × 5 cycles → [94°C for 1 min → 58°C for 1 min → 72°C for 1 min] × 35 cycles.

Subsequently, in the present invention, the amplification product of the bisulfite-treated sample DNA obtained in the amplification step is subjected to ion exchange chromatography. The ion exchange chromatography according to the present invention is preferably anion exchange chromatography. The column packing material for use in the ion exchange chromatography according to the present invention is not particularly limited as long as the packing material is substrate particles having a strong cationic group on the surface. Substrate particles having both a strong cationic group and a weak cationic group on the surface as shown in WO 2012/108516 are preferred.

In the present specification, the strong cationic group means a cationic group which is dissociated in a wide pH range of from 1 to 14. Specifically, the strong cationic group can maintain its dissociated (cationized) state without being influenced by the pH of an aqueous solution.

Examples of the strong cationic group include quaternary ammonium groups. Specific examples thereof include trialkylammonium groups such as a trimethylammonium group, a triethylammonium group, and a dimethylethylammonium group. Examples of the counter ion for the strong cationic group include halide ions such as a chloride ion, a bromide ion, and an iodide ion.

The amount of the strong cationic group present on the surface of the substrate particles is not particularly limited and is preferably 1 µeq/g as the lower limit and 500 µeq/g as the upper limit with respect to the dry weight of the packing material. If the amount of the strong cationic group is less than 1 µeq/g, separation performance may be deteriorated due to weak retention strength. If the amount of the strong cationic group exceeds 500 µeq/g, retention strength may be too strong to easily elute the DNA, resulting in problems such as too long an analysis time.

In the present specification, the weak cationic group means a cationic group having pka of 8 or higher. Specifically, the weak cationic group changes its dissociated state by the influence of the pH of an aqueous solution. Specifically, at pH higher than 8, the proton of the weak cationic group is dissociated so that the ratio of a group having no positive charge increases. On the other hand, at pH lower than 8, the weak cationic group is protonated so that the ratio of a group having positive charge increases.

Examples of the weak cationic group include tertiary amino groups, secondary amino groups, and primary amino groups. Among them, a tertiary amino group is desirable.

The amount of the weak cationic group present on the surface of the substrate particles is not particularly limited and is preferably 0.5 µeq/g as the lower limit and 500 µeq/g as the upper limit with respect to the dry weight of the packing material. If the amount of the weak cationic group is less than 0.5 µeq/g, separation performance may not be improved due to too small an amount. If the amount of the weak cationic group exceeds 500 µeq/g, retention strength may be too strong to easily elute the DNA, resulting in problems such as too long an analysis time, as with the strong cationic group.

The amount of the strong cationic group or the weak cationic group on the surface of the substrate particles can be measured by quantifying a nitrogen atom contained in a group. Examples of the method for quantifying nitrogen include Kjeldahl method. In the case of, for example, substrate particles having the strong cationic group and the weak cationic group, first, nitrogen contained in the strong cationic group is quantified after polymerization of a hydrophobic cross-linked polymer with the strong cationic group. Subsequently, the weak cationic group is introduced to the polymer, and the total amount of nitrogen contained in the strong cationic group and the weak cationic group is quantified. The amount of nitrogen contained in the weak cationic group can be calculated from the determined value. In this way, the amount of the strong cationic group and the amount of the weak cationic group contained in the packing material can be adjusted within the ranges described above on the basis of the quantification values of the nitrogen atoms of the groups.

For example, synthetic polymer fine particles obtained using polymerizable monomers or the like, or inorganic fine particles such as fine silica particles can be used as the substrate particles. Hydrophobic cross-linked polymer particles consisting of a synthetic organic polymer are desirable.

The hydrophobic cross-linked polymer may be any of a hydrophobic cross-linked polymer obtained by copolymerizing at least one hydrophobic cross-linkable monomer and at least one monomer having a reactive functional group, and a hydrophobic cross-linked polymer obtained by copolymerizing at least one hydrophobic cross-linkable monomer, at least one monomer having a reactive functional group, and at least one hydrophobic non-cross-linkable monomer.

The hydrophobic cross-linkable monomer is not particularly limited as long as the monomer has two or more vinyl groups in one molecule. Examples thereof include: di(meth)acrylic acid esters such as ethylene glycol di(meth)acrylate, polyethylene glycol di(meth)acrylate, propylene glycol di(meth)acrylate, and polypropylene glycol di(meth)acrylate; tri(meth)acrylic acid esters such as trimethylol methane tri(meth)acrylate and tetramethylol methane tri(meth)acrylate; tetra(meth)acrylic acid esters; and aromatic compounds such as divinylbenzene, divinyltoluene, divinylxylene, and divinylnaphthalene. In the present specification, the (meth)acrylate means acrylate or methacrylate, and (meth)acryl means acryl or methacryl.

Examples of the monomer having a reactive functional group include glycidyl (meth)acrylate and isocyanatoethyl (meth)acrylate.

The hydrophobic non-cross-linkable monomer is not particularly limited as long as the monomer is a non-cross-linkable polymerizable organic monomer having hydrophobic properties. Examples thereof include: (meth)acrylic acid esters such as methyl (meth)acrylate, ethyl (meth)acrylate, butyl (meth)acrylate, and t-butyl (meth)acrylate; and styrene monomers such as styrene and methylstyrene.

When the hydrophobic cross-linked polymer is obtained by copolymerizing the hydrophobic cross-linkable monomer and the monomer having a reactive functional group, the content ratio of a segment derived from the hydrophobic cross-linkable monomer in the hydrophobic cross-linked polymer is preferably 10 wt% as the lower limit, more preferably 20 wt% as the lower limit.

The packing material for the ion exchange chromatography used in the present invention preferably has a polymer layer having the strong cationic group and the weak cationic group on the surface of the substrate particles. For the polymer having the strong cationic group and the weak cationic group, it is preferred that the strong cationic group and the weak cationic group should be respectively derived from independent monomers. Specifically, the packing material for the ion exchange chromatography used in the present invention is preferably a packing material in which the weak cationic group is introduced in the surface of coated polymer particles consisting of the hydrophobic cross-linked polymer particles and a layer of a hydrophilic polymer having the strong cationic group copolymerized at the surface of the hydrophobic cross-linked polymer particles.

The hydrophilic polymer having the strong cationic group is formed from hydrophilic monomers having the strong cationic group and can contain a segment derived from one or more hydrophilic monomers having the strong cationic group. Specifically, examples of the method for producing the hydrophilic polymer having the strong cationic group include a method which involves homopolymerizing a hydrophilic monomer having the strong cationic group, a method which involves copolymerizing two or more hydrophilic monomers each having the strong cationic group, and a method which involves copolymerizing a hydrophilic monomer having the strong cationic group and a hydrophilic monomer having no strong cationic group.

The hydrophilic monomer having the strong cationic group preferably has a quaternary ammonium group. Specific examples thereof include ethyl methacrylate trimethylammonium chloride, ethyl methacrylate triethylammonium chloride, ethyl methacrylate dimethylethylammonium chloride, ethyl methacrylate dimethylbenzylammonium chloride, ethyl acrylate dimethylbenzylammonium chloride, ethyl acrylate trimethylammonium chloride, ethyl acrylate triethylammonium chloride, ethyl acrylate dimethylethylammonium chloride, acrylamide ethyltrimethylammonium chloride, acrylamide ethyltriethylammonium chloride, and acrylamide ethyl dimethylethylammonium chloride.

Examples of the method for forming the layer of a hydrophilic polymer having the strong cationic group on the surface of the hydrophobic cross-linked polymer include a method of copolymerizing a hydrophilic monomer having the strong cationic group onto the surface of the hydrophobic cross-linked polymer.

A method known in the art can be used as a method for introducing the weak cationic group to the surface of the coated polymer particles. Specifically, examples of the method for introducing a tertiary amino group as the weak cationic group include: a method which involves copolymerizing the hydrophilic monomer having the strong cationic group at the surface of the hydrophobic cross-linked polymer particles having a segment derived from a monomer having a glycidyl group, and subsequently reacting the glycidyl group with a reagent having a tertiary amino group; a method which involves copolymerizing the hydrophilic monomer having the strong cationic group at the surface of the hydrophobic cross-linked polymer particles having a segment derived from a monomer having an isocyanate group, and subsequently reacting the isocyanate group with a reagent having a tertiary amino group; a method which involves copolymerizing the hydrophilic monomer having the strong cationic group and a monomer having a tertiary amino group at the surface of the hydrophobic cross-linked polymer particles; a method which involves introducing a tertiary amino group to the surface of the coated polymer particles having a hydrophilic polymer layer having the strong cationic group using a silane coupling agent having the tertiary amino group; a method which involves copolymerizing the hydrophilic monomer having the strong cationic group at the surface of the hydrophobic cross-linked polymer particles having a segment derived from a monomer having a carboxy group, and subsequently condensing the carboxy group with a reagent having a tertiary amino group using carbodiimide; and a method which involves copolymerizing the hydrophilic monomer having the strong cationic group at the surface of the hydrophobic cross-linked polymer particles having a segment derived from a monomer having an ester bond, hydrolyzing the ester bond moiety, and then condensing a carboxy group formed by the hydrolysis with a reagent having a tertiary amino group using carbodiimide. Among them, the method which involves copolymerizing the hydrophilic monomer having the strong cationic group at the surface of the hydrophobic cross-linked polymer particles having a segment derived from a monomer having a glycidyl group, and subsequently reacting the glycidyl group with a reagent having a tertiary amino group, or the method which involves copolymerizing the hydrophilic monomer having the strong cationic group at the surface of the hydrophobic cross-linked polymer particles having a segment derived from a monomer having an isocyanate group, and subsequently reacting the isocyanate group with a reagent having a tertiary amino group, is preferred.

The reagent having a tertiary amino group which is reacted with the reactive functional group such as a glycidyl group or an isocyanate group is not particularly limited as long as the reagent has a functional group reactable with the tertiary amino group and the reactive functional group. Examples of the functional group reactable with the reactive functional group include primary amino groups and a hydroxy group. Among others, a group having a terminal primary amino group is preferred. Specific examples of the reagent having a tertiary amino group which has the functional group include N,N-dimethylaminomethylamine, N,N-dimethylaminoethylamine, N,N-dimethylaminopropylamine, N,N-dimethylaminobutylamine, N,N-diethylaminoethylamine, N,N-diethylaminopropylamine, N,N-diethylaminobutylamine, N,N-diethylaminopentylamine, N,N-diethylaminohexylamine, N,N-dipropylaminobutylamine, and N,N-dibutylaminopropylamine.

For the relative positional relationship between the strong cationic group (preferably, a quaternary ammonium salt) and the weak cationic group (preferably, a tertiary amino group), it is preferred that the strong cationic group should be positioned more distant than the weak cationic group from the surface of the substrate particles, i.e., positioned on the outer side of the weak cationic group. Preferably, for example, the weak cationic group is located within 30 angstroms from the surface of the substrate particles, and the strong cationic group is located within 300 angstroms from the surface of the substrate particles and on the outer side of the weak cationic group.

The average particle size of the substrate particles which are used as the packing material for the ion exchange chromatography used in the present invention is not particularly limited and is preferably 0.1 µm as the lower limit and 20 µm as the upper limit. If the average particle size is less than 0.1 µm, poor separation may occur due to too high a pressure inside the column. If the average particle size exceeds 20 µm, poor separation may occur due to too large a dead volume inside the column. In the present specification, the average particle size refers to a volume-average particle size and can be measured using a particle size distribution measurement apparatus (e.g., AccuSizer 780, manufactured by Particle Sizing Systems).

The sample injection volume to the ion exchange chromatography column is not particularly limited and can be appropriately adjusted according to the ion exchange capacity of the column and the sample concentration. The flow rate is preferably from 0.1 mL/min to 3.0 mL/min, more preferably from 0.5 mL/min to 1.5 mL/min. At a slower flow rate, improved separation can be expected. Too slow a flow rate might require a long time for analysis or incur reduction in separation performance due to broader peaks. On the other hand, a faster flow rate is advantageous in terms of reduction in analysis time, but incurs reduction in separation performance due to peak compression. Accordingly, it is desirable to set the flow rate to within the range described above, though this parameter is appropriately adjusted according to the performance of the column. The retention time of each sample can be predetermined by a preliminary experiment on each sample. A flowing method known in the art, such as linear gradient elution method or stepwise elution method can be used. The flowing method according to the present invention is preferably linear gradient elution method. The amplitude of the gradient can be appropriately adjusted within a range of the eluent for use in elution from 0% to 100% according to the separation performance of the column and the characteristics of the analyte (here, the amplification product DNA obtained in the amplification step).

Conditions known in the art can be used for the composition of an eluent for use in the ion exchange chromatography according to the present invention.

The buffer solution for use in the eluent is preferably a buffer solution containing a salt compound known in the art, or an organic solvent. Specific examples thereof include a tris-HCl buffer solution, a TE buffer solution consisting of tris and EDTA, and a TBA buffer solution consisting of tris, boric acid, and EDTA.

The pH of the eluent is not particularly limited and is preferably 5 as the lower limit and 10 as the upper limit. At the pH set within this range, the weak cationic group is considered to also work effectively as an ion exchange group (anion exchange group). The pH of the eluent is more preferably 6 as the lower limit and 9 as the upper limit.

Examples of the salt contained in the eluent include: salts consisting of a halide and an alkali metal, such as sodium chloride, potassium chloride, sodium bromide, and potassium bromide; and salts consisting of a halide and an alkaline earth metal, such as calcium chloride, calcium bromide, magnesium chloride, and magnesium bromide; and inorganic acid salts such as sodium perchlorate, potassium perchlorate, sodium sulfate, potassium sulfate, ammonium sulfate, sodium nitrate, and potassium nitrate. Alternatively, an organic acid salt such as sodium acetate, potassium acetate, sodium succinate, or potassium succinate may be used. Any one of these salts may be used alone or, two or more thereof may be used in combination.

The salt concentration of the eluent can be appropriately adjusted according to analysis conditions and is preferably 10 mmol/L as the lower limit and 2,000 mmol/L as the upper limit, more preferably 100 mmol/L as the lower limit and 1,500 mmol/L as the upper limit.

The eluent further contains an anti-chaotropic ion for further enhancing separation performance. The anti-chaotropic ion has properties opposite to those of a chaotropic ion and works to stabilize a hydrated structure. Therefore, the anti-chaotropic ion is effective for strengthening the hydrophobic interaction between the packing material and a nucleic acid molecule. The main interaction of the ion exchange chromatography used in the present invention is electrostatic interaction. Separation performance is enhanced through the use of the work of the hydrophobic interaction in addition thereto.

Examples of the anti-chaotropic ion contained in the eluent include a phosphate ion (PO₄³⁻), a sulfate ion (SO₄²⁻), an ammonium ion (NH₄⁺), a potassium ion (K⁺), and a sodium ion (Na⁺). Among combinations of these ions, a sulfate ion and an ammonium ion are preferably used. Any one of these anti-chaotropic ions may be used alone, or two or more thereof may be used in combination. Some of the anti-chaotropic ions may comprise a salt contained in the eluent or a component of the buffer solution. Such a component is suitable for the present invention, because the component possesses both of properties or buffering ability as the salt contained in the eluent and properties as the anti-chaotropic ion.

The concentration of the anti-chaotropic ion contained in the eluent can be appropriately adjusted according to an analyte and is desirably 2,000 mmol/L or lower in terms of anti-chaotropic salt. Specific examples of such a method can include a method which involves performing gradient elution at anti-chaotropic salt concentrations ranging from 0 to 2,000 mmol/L. Thus, the concentration of the anti-chaotropic salt at the start of chromatography analysis does not have to be 0 mmol/L, and the concentration of the anti-chaotropic salt at the completion of analysis does not have to be 2,000 mmol/L. The gradient elution method may be a low-pressure gradient method or may be a high-pressure gradient method. The method preferably involves performing elution while the concentration is precisely adjusted by the high-pressure gradient method.

The anti-chaotropic ion may be added to only one eluent for use in elution or may be added to a plurality of eluents. Also, the anti-chaotropic ion may play a role both in the effect of enhancing the hydrophobic interaction between the packing material and the DNA or the buffering ability and in the effect of eluting the amplification product DNA from the column.

In the ion exchange chromatography used in the present invention, the column temperature for analyzing the amplification product DNA obtained in the amplification step is preferably 30°C or higher, more preferably 40°C or higher, further preferably 45°C or higher, still further preferably 60°C or higher. If the column temperature is lower than 30°C, the hydrophobic interaction between the packing material and the amplification product DNA weakens and it becomes difficult to obtain the desired separating effect. On the other hand, as the column temperature in the ion exchange chromatography is higher, the amplification product derived from methylated DNA and the amplification product derived from unmethylated DNA are more clearly separated. When the column temperature is 60°C or higher, the amplification product derived from methylated DNA and the amplification product derived from unmethylated DNA differ more largely in the retention time of a peak of a chromatography detection signal and respectively exhibit more clear peaks. Therefore, the detection of DNA methylation and the risk evaluation of HCC can be attained more accurately.

On the other hand, a column temperature of 90°C or higher in the ion exchange chromatography is not preferred for the analysis because two strands of the amplification product DNA are dissociated. A column temperature of 100°C or higher is not preferred for the analysis because the eluent might be boiled. Thus, the column temperature for analyzing the amplification product DNA by the ion exchange chromatography used in the present invention can be 30°C or higher and lower than 90°C and is preferably 40°C or higher and lower than 90°C, more preferably 45°C or higher and lower than 90°C, further preferably 55°C or higher and lower than 90°C, further preferably 60°C or higher and lower than 90°C, further preferably 55°C or higher and 85°C or lower, particularly preferably 60°C or higher and 85°C or lower.

The methylation of the CpG site of an exon region of MGRN1 gene can be used as an index for evaluating a risk of HCC (Patent Literature 1; JP-A-2010-148426; Int J Cancer, 2009, 125, 2854-2862; and Int J Cancer, 2011, 129, 1170-1179). Thus, the risk of HCC can be evaluated on the basis of the methylation level of the sample DNA comprising a CpG site of an exon region of MGRN1 gene. In the present invention, the methylation level of the sample DNA appears as the difference in the peak shape of a detection signal obtained by the ion exchange chromatography analysis.

The treatment of DNA with bisulfite converts unmethylated BR>Vtosine in the DNA to uracil, while leaving methylated cytosine unaltered. The amplification of the bisulfite-treated DNA further replaces uracil derived from the unmethylated cytosine with thymine and therefore results in the difference in the abundance ratios of cytosine and thymine between methylated DNA and unmethylated DNA. Thus, the amplified DNA has a distinctive sequence according to the methylation rate. When DNA having a distinctive sequence is subjected to ion exchange chromatography a chromatogram showing a distinctive signal is obtained according to the distinctive sequence.

More specifically, in the ion exchange chromatography analysis, the amplitude of the DNA methylation level is reflected to the retention time of a peak of a detection signal. In the ion exchange chromatography analysis, for example, 100% methylated DNA and unmethylated DNA can be detected as their respective independent peaks, and the peak of the methylated DNA typically appears at a retention time shorter than that of the peak of the unmethylated DNA (see Patent Literature 2). Thus, when the methylated DNA is absent or is present in a very small amount in the sample DNA, the peak of a detection signal of the chromatography has a unimodal shape where only the peak of the unmethylated DNA appears. On the other hand, as the ratio of the methylated DNA increases in the sample DNA, the peak of the methylated DNA appears separately at a shorter retention time, and the detection signal has a bimodal shape having the peak of the methylated DNA and the peak of the unmethylated DNA. In this respect, if the proportion of the methylated DNA in the sample DNA is low, the second modal peak of the methylated DNA can appear as a bulge or a shoulder on the slope of the first modal peak of the unmethylated DNA. As the proportion of the methylated DNA increases, the second modal peak can appear more clearly. As the proportion of the methylated DNA further increases so that the methylated DNA is predominant, the first modal peak is smaller in size and can appear as a bulge or a shoulder on the slope of the second modal peak. As the DNA methylation progresses to almost completely methylate the sample DNA, the peak of the detection signal has a unimodal shape where only the peak of the methylated DNA appears. Thus, the peak shape of the signal obtained by the ion exchange chromatography analysis of the sample DNA indicates the methylation level of the sample DNA.

The peak shape of the signal obtained by the chromatography analysis of the sample DNA reflects the DNA methylation level thereof and has an association with a risk of HCC. Thus, the risk of development of HCC in the subject can be evaluated on the basis of the peak shape of the detection signal of the ion exchange chromatography according to the present invention. More components of the peak of methylated DNA in the peak of the detection signal mean that malignant transformation is more progressive in the liver tissue, and the subject has a higher risk of HCC. On the other hand, more components of the peak of unmethylated DNA in the peak of the detection signal mean that malignant transformation is less progressive in the liver tissue, and the subject has a lower risk of HCC.

In a preferred embodiment of the present invention, the peak shape of the detection signal of the ion exchange chromatography is determined whether it is unimodal or bimodal. In this context, the term "unimodal" means that the peak of methylated DNA appears as a unimodal peak. The determination of the peak shape as being unimodal or bimodal may be made from the shape of a curve of the plot of the detection signal against a retention time, or may be made on the basis of the derivative value of the detection signal for more accurate analysis. For example, when the plot of the first derivative value of the detection signal against a retention time is represented by a curve having two or more maximums, the peak shape of the detection signal is determined as being bimodal.

In one embodiment of the present invention, whether or not the sample DNA is DNA obtained from a subject having a high risk of development of HCC is determined on the basis of a peak shape of a detection signal of the chromatography. Preferably, the sample DNA is determined as being DNA obtained from a subject having a high risk of development of HCC when the peak is unimodal, and determined as being DNA obtained from a subject having a low risk of development of HCC when the peak is bimodal.

In another embodiment of the present invention, whether or not the subject is a subject having a high risk of development of HCC is determined on the basis of a peak shape of a detection signal of the chromatography. Preferably, the subject is determined as being a subject having a high risk of development of HCC when the peak is unimodal, and determined as being a subject having a low risk of development of HCC when the peak is bimodal.

Examples of the method for determining the presence or absence of the peak of the detection signal obtained by the chromatography include peak detection using existing data processing software, for example, LCsolution (Shimadzu Corp.), GRAMS/AI (Thermo Fisher Scientific, Inc.), or Igor Pro (WaveMetrics). The method for determining the presence or absence of the peak using LCsolution will be described as an example.
Specifically, a retention time zone in which a peak is to be detected is first designated. Next, various parameters are set in order to remove unnecessary peaks such as noise. Examples of such settings include setting of the parameter "WIDTH" to larger than the half widths of unnecessary peaks, setting of the parameter "SLOPE" to larger than the leading slopes of unnecessary peaks, and changing of the parameter "DRIFT" setting to select either vertical partitioning or baseline partitioning of peaks with a low degree of separation. The values of these parameters can be set to appropriate values according to a chromatogram because the obtained chromatogram differs depending on analysis conditions, the type or the amount of a DNA to be analyzed, etc.

The derivative value of the detection signal can be automatically calculated using the data processing software mentioned above. Alternatively, the derivative value can be calculated using spreadsheet software (Microsoft(R) Excel(R), for example), etc.

The time range in which the derivative value of the detection signal of the chromatography is calculated can be appropriately set. Also, the time range in which the maximum of the first derivative value is detected can be appropriately set. The maximum of the first derivative value can be detected, for example, in a time range from the start to rise to the end of a peak curve of the detection signal.

In the present invention, the detection signal of the chromatography of the sample DNA can be compared with a detection signal as to control DNA. DNA having the same sequence as that of the sample DNA and a known methylation level (e.g., 0% or 100%) is used as the control DNA.

For example, the detection signal as to 0% methylated control (negative control) can be acquired by performing bisulfite treatment, nucleic acid amplification, and ion exchange chromatography according to the procedures mentioned above using DNA having the same sequence, albeit not methylated, as that of the sample DNA. For example, the detection signal as to 100% methylated control (positive control) can be acquired by performing bisulfite treatment, nucleic acid amplification, and ion exchange chromatography according to the procedures mentioned above using 100% methylated DNA having the same sequence as that of the sample DNA. Alternatively, the detection signals as to the negative control and the positive control can be acquired by synthesizing DNA sequences by the bisulfite treatment and nucleic acid amplification of sample DNA having a methylation rate of 0% and 100%, respectively, and subsequently subjecting the resulting products to ion exchange chromatography.

The peak of unmethylated DNA contained in the sample DNA appears as a peak having a retention time equivalent to that of the negative control. On the other hand, the retention time of a peak derived from methylated DNA contained in the sample DNA should be shifted to a peak from the positive control according to the methylation level thereof. The retention time of the peak from the sample DNA can be compared with that from the negative control or the positive control to confirm that the signal from the sample DNA containing unmethylated DNA or methylated DNA has been correctly obtained. This procedure can be effective for preventing determination errors attributed to mistakes in the operation of sample DNA preparation or subsequent treatment steps. However, this procedure is not necessarily required, particularly, when a clear unimodal or bimodal peak is obtained from the sample DNA.

The method for evaluating a risk of HCC according to the present invention employs ion exchange chromatography in the detection of DNA methylation and is therefore a very convenient and rapid method compared with a conventional method for detecting DNA methylation by pyrosequencing or the like (e.g., Patent Literature 1). Further surprisingly, the present invention enables a risk of HCC to be evaluated with remarkably high sensitivity and specificity by using the methylation of a CpG site of an exon region of MGRN1 gene as an index. As shown in Examples mentioned later, in the risk evaluation of HCC using the detection of DNA methylation by ion exchange chromatography, DNA comprising a CpG site of an exon region of MGRN1 gene (Liv25 region; SEQ ID NO: 1) was selected as target DNA for the detection of methylation. In this case, its sensitivity and specificity were remarkably improved as compared with DNA comprising its neighboring CpG site (CpG site of an intron region of the MGRN1 gene) (Liv27 region; SEQ ID NO: 2), or DNA comprising a CpG site of a different gene (KDM4B) (Liv28 region; SEQ ID NO: 3). The neighboring CpG site and the CpG site of the KDM4B gene are sites which attained 100% sensitivity and specificity in the risk evaluation of HCC by pyrosequencing (see Patent Literature 1, Table 4). However, both of these two sites exhibited considerable reduction in sensitivity or specificity in the evaluation by the ion exchange chromatography. By contrast, the DNA comprising a CpG site of an exon region of MGRN1 gene attained very high sensitivity and specificity equivalent to those of the method using pyrosequencing, even in the risk evaluation of HCC by the ion exchange chromatography according to the present invention. Thus, the risk evaluation method of HCC by the ion exchange chromatography according to the present invention is an excellent method which can achieve all of rapidness, convenience, and very high sensitivity and specificity.

### Examples

Hereinafter, the present invention will be described in detail with reference to Examples. However, the present invention is not intended to be limited by Examples given below.

### [Method]

### (1) Patient and specimen

Specimens were obtained as 36 samples of noncancerous liver tissues (N group) excised from patients having a history of hepatitis virus (HBV or HCV) infection and having HCC, and 36 samples of healthy liver tissues (C group) excised from patients neither having a history of hepatitis virus infection nor having HCC.

### (2) Preparation of DNA

Fresh frozen tissue samples obtained from the patients were each treated with phenol-chloroform and subsequently dialyzed to extract high-molecular-weight DNA (see Sambrook, J. et al., Molecular Cloning: A Laboratory Manual, 3rd edition, Cold Spring Harbor Laboratory Press, NY, p. 6.14-6.15). 500 ng of the obtained DNA was treated with bisulfite using EZ DNA Methylation-Gold (TM) kit (manufactured by Zymo Research Corp.).

### (3) PCR amplification

The bisulfite-treated genomic DNA obtained in the preceding step (2) was amplified by PCR. In the PCR, the following three different regions comprising a CpG site in the genomic DNA were amplified.
Liv25 region: DNA region comprising a CpG site of an exon region of MGRN1 gene
Liv27 region: DNA region comprising a neighboring CpG site (CpG site of an intron region of the MGRN1 gene) of the Liv25 region
Liv28 region: DNA region comprising a CpG site of a different gene (KDM4B)

Table 1 shows the nucleotide sequences of the amplified regions (sequences before the bisulfite treatment) and primers used in the amplification. As for each of the regions, DNA having a methylation rate of 0% (negative control) and DNA having a methylation rate of 100% (positive control) were prepared so as to have the same sequence thereas, and treated with bisulfite and amplified by PCR according to the same procedures as above.

Composition of a PCR reaction solution (50 µL) : 75 ng of template DNA, 1 × QIAGEN PCR buffer (manufactured by Qiagen N.V.), 200 µmol/L dNTP Mix (manufactured by Toyobo Co., Ltd.), 2.5 U HotStartTaq Plus DNA Polymerase (manufactured by Qiagen N.V.), and 0.2 µmol/L forward and reverse primers. The PCR conditions were as follows:
Liv25: [94°C for 1 min → 64°C for 1 min → 72°C for 1 min] × 5 cycles → [94°C for 1 min → 62°C for 1 min → 72°C for 1 min] × 5 cycles → [94°C for 1 min → 60°C for 1 min → 72°C for 1 min] × 5 cycles → [94°C for 1 min → 58°C for 1 min → 72°C for 1 min] × 35 cycles
Liv27: [94°C for 1 min → 56°C for 1 min → 72°C for 1 min] × 50 cycles
Liv28: [94°C for 1 min → 56°C for 1 min → 72°C for 1 min] × 50 cycles

After the completion of the PCR, a mixture of 5 µL of the reaction solution with 1 µL of a loading dye solution was applied to a 3% agarose gel supplemented with ethidium bromide in advance, and electrophoresed to confirm that the PCR amplification product of interest was obtained.

**[Table 1]**

| Amplified region | Sequence before bisulfite treatment) | The number of CpG sites | Primer | Region | SEQ ID NO |
|---|---|---|---|---|---|
| Liv25 | tactggagaagcggctgtgtccacatagccacctccac ggcccctcagcctggcaggggaggaggcagcctccgcag atgggccgctgacccgctgcctttctctccaccgcctg ggtaggtacaaagacgatgccgacagccccaccgagga cggcgacaagccccgggtgctctacagcctggagttcac cttcgacgccgatgcccgcgtggccatcaccatctactg ccaggcatcggaggagttcctgaacggcagggcagtgtg agtcccgcgg (SEQ ID NO: 1) | 18 | Forward | tattggagaagagggttgtgtttatat | 4 |
| | | | Reverse | cccccaaactcacactaccctac | 5 |
| Liv27 | agctggccctgcgggaaagcagctcccctgaggtgaggc ccccccggggaagctttgcgcacccgcccgggccagccc tcctccagcttctgtcgctggaatcagaccccatcccac tgcccgcctgggcgcccccgtgcttgttggctcttgctg agctgcgcggctccttagcctcggtctgtggaggcagca ccccctggtggag (SEQ ID NO: 2) | 11 | Forward | agttggttttgagggaaagtagt | 6 |
| | | | Reverse | ctccaccaaaaaatactacctcc | 7 |
| Liv28 | caggccctacagttaggagggcagggcgtggcgctgcag gcctgtgtgcagtgtggtggacctgcccctgcccgggag gtgccgggtgcagcgtgggcgctgctgtggcggctgag ttccaggaccttctcctggctccctgtgcacgtcgcagc gaggccgtgtgggggtgtgtgtgaatgtgcacgtgtgcg cgcgcatgtgagtgcgcacgtgagtgagtgtgagtatgc atgcgtgtgtgtgcccctgtgtcaccggctctgcttgt gactgcggaacctgctggtgctccgc (SEQ ID NO: 3) | 19 | Forward | taggttttatagttaggagggtagg | 8 |
| | | | Reverse | cccaaacacccaacaaattc | 9 |

### (4) Preparation of anion exchange column

In a reactor equipped with a stirrer, a mixture of 200 g of tetraethylene glycol dimethacrylate (manufactured by Shin-Nakamura Chemical Co., Ltd.), 100 g of triethylene glycol dimethacrylate (manufactured by Shin-Nakamura Chemical Co., Ltd.), 100 g of glycidyl methacrylate (manufactured by Wako Pure Chemical Industries, Ltd.), and 1.0 g of benzoyl peroxide (manufactured by Kishida Chemical Co., Ltd.) was added to 2,000 mL of an aqueous solution containing 3 wt% of polyvinyl alcohol (manufactured by The Nippon Synthetic Industry Co., Ltd.). The reaction mixture was heated with stirring and polymerized at 80°C for 1 hour in the nitrogen atmosphere. Next, 100 g of ethyl methacrylate trimethylammonium chloride (manufactured by Wako Pure Chemical Industries, Ltd.) was dissolved in ion exchange water as the hydrophilic monomer having the strong cationic group. This solution was added to the reactor mentioned above. Similarly, the reaction mixture was polymerized with stirring at 80°C for 2 hours in the nitrogen atmosphere. The obtained polymer composition was washed with water and acetone to obtain coated polymer particles having, on the surface, a hydrophilic polymer layer having a quaternary ammonium group. The obtained coated polymer particles were found to have an average particle size of 10 µm by measurement using a particle size distribution measurement apparatus (AccuSizer 780, manufactured by Particle Sizing Systems).

10 g of the obtained coated polymer particles was dispersed in 100 mL of ion exchange water to prepare pre-reaction slurry. Subsequently, 10 mL of N,N-dimethylaminopropylamine (manufactured by Wako Pure Chemical Industries, Ltd.), a reagent having a weak cationic group, was added to this slurry with stirring, and the mixture was reacted at 70°C for 4 hours. After the completion of the reaction, the supernatant was removed using a centrifuge (manufactured by Hitachi, Ltd., "Himac CR20G"), and the residue was washed with ion exchange water. After the washing, the supernatant was removed using a centrifuge. This washing with ion exchange water was further repeated four times to obtain a packing material for ion exchange chromatography having a quaternary ammonium group and a tertiary amino group on the surface of the substrate particles.

A stainless column (column size: inside diameter 4.6 mm × length 20 mm) of a liquid chromatography system was packed with the obtained packing material for ion exchange chromatography.

### (5) HPLC analysis

The anion exchange column prepared in the step (4) was used in ion exchange chromatography under the following conditions to separate and detect each PCR amplification product obtained in the preceding step (3).
System: LC-20A series (manufactured by Shimadzu Corp.)
Eluent: eluent A: 25 mmol/L tris-HCl buffer solution (pH 7.5)
eluent B: 25 mmol/L tris-HCl buffer solution (pH 7.5) + 1 mol/L ammonium sulfate
Analysis time: 15 min
Elution method: the mixing ratio of eluent B was linearly increased under the following gradient conditions:
   0 min (40% eluent B) → 10 min (100% eluent B)
   Specimen: the PCR amplification product obtained in the step (2)
   Flow rate: 1.0 mL/min
   Detection wavelength: 260 nm
   Sample injection volume: 5 µL, or 2 µL for the negative control and the positive control
   Column temperature: 70°C

### (6) Risk evaluation of HCC by methylation analysis of CpG site of MGRN1 gene exon region using chromatography

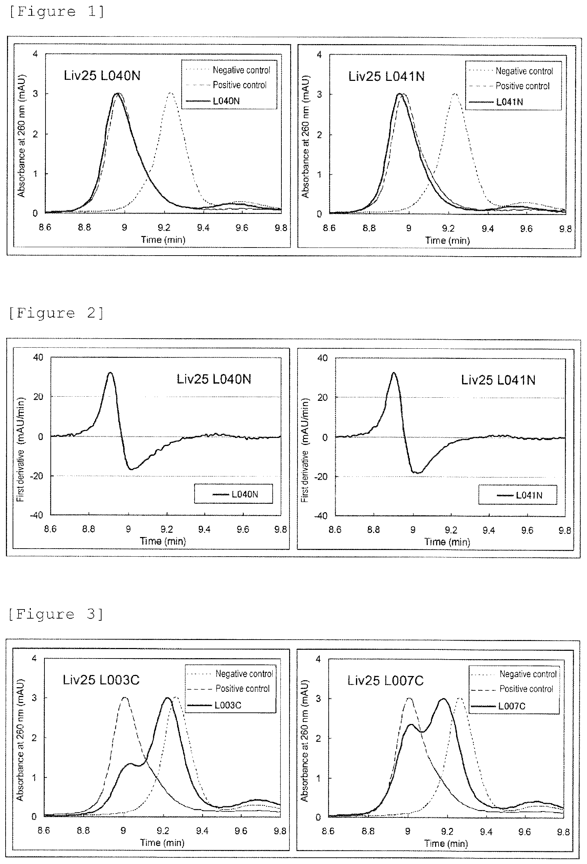

Among HPLC chromatograms obtained from the DNA comprising a CpG site of an exon region of MGRN1 gene (Liv25 region) in the noncancerous liver tissue samples (N group) derived from the HCC patients, typical examples are shown in Figure 1. Each diagram of Figure 1 also displays overlaid chromatograms of the unmethylated DNA (negative control) and the 100% methylated DNA (positive control). In these N group samples, only a unimodal peak overlapping with the peak from the positive control appeared. The first derivative values (Figure 2) of these data had a curve having one maximum. Samples with a non-unimodal peak such as a shoulder peak in chromatograms were able to be determined from a curve of a first derivative value having two maximums (not shown).

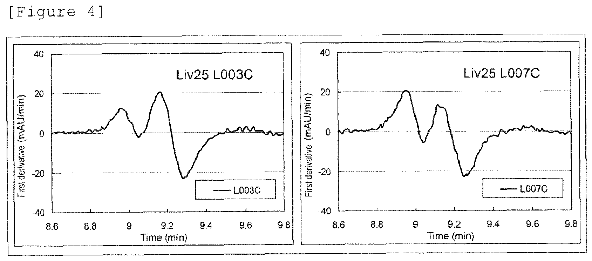

Among HPLC chromatograms obtained from the Liv25 region in the healthy liver tissue samples (C group), typical examples are shown in Figure 3. Figure 3 also displays overlaid chromatograms of the unmethylated DNA (negative control) and the 100% methylated DNA (positive control). In these C group samples, a bimodal peak appeared. From the comparison between the negative control and the positive control, the bimodal peak was presumed to comprise the peak of the 100% methylated DNA and the peak of DNA having a lower methylation rate. The first derivative values (Figure 4) of these data had a curve having two maximums.

In conclusion, the elevation of the DNA methylation level of the DNA comprising a CpG site of an exon region of MGRN1 gene (Liv25 region) in the noncancerous liver tissues of the HCC patients was able to be detected by ion exchange chromatography analysis. The peak shape of the chromatography analysis on the Liv25 region differed clearly between the N group and the C group and permitted the discrimination of the tissues of the HCC patients from the healthy tissues.

### (7) Risk evaluation of HCC by methylation analysis of other regions

HPLC chromatograms obtained from the Liv27 region tended to have a unimodal peak for the N group and a bimodal peak for the C group, as in the Liv25 region. On the other hand, HPLC chromatograms obtained from the Liv28 region tended to have a bimodal peak for the N group and a unimodal peak for the C group. However, peaks inconsistent with the tendencies were not infrequently obtained from the Liv27 region and the Liv28 region both for the N group and for the C group, suggesting that the tissues of HCC patients cannot be accurately discriminated from healthy tissues only on the basis of a peak shape.

### (8) Comparison of sensitivity and specificity of risk evaluation of HCC among regions

The risk of HCC was evaluated using the chromatograms from the Liv25 region, the Liv27 region and the Liv28 region, and the sensitivity (positive agreement rate) and specificity (negative agreement rate) thereof were calculated. For the Liv25 and Liv27 regions, samples from which a chromatogram having a unimodal peak was obtained were determined as being positive for the risk of HCC, and samples from which a chromatogram having a bimodal peak was obtained were determined as being negative for the risk of HCC. For the Liv28 region, samples from which a chromatogram having a bimodal peak was obtained were determined as being positive for the risk of HCC, and samples from which a chromatogram having a unimodal peak was obtained were determined as being negative for the risk of HCC. Table 2 shows the number of samples determined as being positive or negative for the risk of HCC in the N group and the C group, and the sensitivity and specificity of evaluation, as to each of the regions. The evaluation using the Liv25 region had both very high sensitivity and specificity and was remarkably highly accurate as compared with the other regions.

**[Table 2]**

| **Liv25** | | Presence or absence of HCC | | | |
|---|---|---|---|---|---|
| | | Present (N group) | Absent (C group) | | |
| Risk evaluation of HCC | Positive | 34 | 0 | Sensitivity: | 94.4 % |
| | Negative | 2 | 36 | Specificity: | 100.0 % |
| | | | | | |

| **Liv27** | | Presence or absence of HCC | | | |
|---|---|---|---|---|---|
| | | Present (N group) | Absent (C group) | | |
| Risk evaluation of HCC | Positive | 28 | 5 | Sensitivity: | 77.8 % |
| | Negative | 8 | 31 | Specificity: | 86.1 % |
| | | | | | |

| **Liv28** | | Presence or absence of HCC | | | |
|---|---|---|---|---|---|
| | | Present (N group) | Absent (C qroup) | | |
| Risk evaluation of HCC | Positive | 34 | 29 | Sensitivity: | 94.4 % |
| | Negative | 2 | 7 | Specificity: | 19.4 % |

## Claims

1. A method for evaluating a risk of hepatocellular carcinoma, comprising:
(1) amplifying bisulfite-treated DNA derived from a liver tissue of a subject, wherein the DNA comprises a CpG site of an exon region of MGRN1 gene;
(2) subjecting the obtained amplification product to ion exchange chromatography; and
(3) determining whether or not the DNA is DNA obtained from a subject having a high risk of development of hepatocellular carcinoma on the basis of a peak shape of a detection signal of the chromatography.

2. A method for evaluating a risk of hepatocellular carcinoma, comprising:
(1) amplifying bisulfite-treated DNA derived from a liver tissue of a subject, wherein the DNA comprises a CpG site of an exon region of MGRN1 gene;
(2) subjecting the obtained amplification product to ion exchange chromatography; and
(3) determining whether or not the subject has a high risk of development of hepatocellular carcinoma on the basis of a peak shape of a detection signal of the chromatography.

3. The method according to claim 1 or 2, wherein the DNA comprises DNA consisting of the nucleotide sequence represented by SEQ ID NO: 1, or a nucleotide sequence having at least 95% identity to the sequence.

4. The method according to any one of claims 1 to 3, wherein in the step (3), when the peak shape of a detection signal is a unimodal peak of methylated DNA, the DNA is selected as DNA obtained from a subject having a high risk of development of hepatocellular carcinoma, and when the peak shape of a detection signal is bimodal, the DNA is selected as DNA obtained from a subject having a low risk of development of hepatocellular carcinoma.

5. The method according to claim 4, wherein
the step (3) comprises comparing the retention time of the peak of the detection signal with the retention time of a peak of a detection signal of a positive control or a negative control to confirm that the peak of the methylated DNA has been obtained, wherein
the detection signal of the positive control is obtained by subjecting, to ion exchange chromatography, DNA obtained by the bisulfite treatment and amplification of 100% methylated DNA consisting of the same sequence as that of the DNA derived from a liver tissue of a subject, and
the detection signal of the negative control is obtained by subjecting, to ion exchange chromatography, DNA obtained by the bisulfite treatment and amplification of unmethylated DNA consisting of the same sequence as that of the DNA derived from a liver tissue of a subject.

6. The method according to any one of claims 1 to 5, wherein the ion exchange chromatography is anion exchange chromatography.
